# EUROPÄISCHE PATENTSCHRIFT

(11) **EP 4 333 934 B1**
(45) Veröffentlichungstag und Bekanntmachung des Hinweises auf die Patenterteilung: **31.12.2025**
(21) Anmeldenummer: 22722853.3
(22) Anmeldetag: 29.04.2022
(51) Int. Cl.: A61M 5/165, A61M 5/38

(54) **INFUSIONSFILTER, UND INFUSIONSSET MIT INFUSIONSFILTER**
INFUSION FILTER, AND INFUSION SET WITH INFUSION FILTER
FILTRE DE PERFUSION ET ENSEMBLE DE PERFUSION AVEC FILTRE DE PERFUSION

(30) Priorität: 06.05.2021 DE 102021111888
(43) Veröffentlichungstag der Anmeldung: 13.03.2024
(73) Patentinhaber: B. Braun Melsungen AG, 34212 Melsungen (DE)
(72) Erfinder: ZERBES, Michael, 34590 Wabern (DE); FREITAG, Claudia, 34281 Gudensberg (DE); KATERKAMP, Andreas, 34212 Melsungen (DE)
(74) Vertreter: Winter, Brandl - Partnerschaft mbB
(86) Internationale Anmeldenummer: PCT/EP2022/061519
(87) Internationale Veröffentlichungsnummer: WO 2022/233731

(56) Entgegenhaltungen:
- WO-A1-2020/080889
- CA-A1- 2 167 932
- DE-A1- 2 627 169
- US-A1- 2013 066 272

## Beschreibung

Die Offenbarung betrifft einen Infusionsfilter/Luftabscheider für eine Infusionsleitung eines Infusionssets mit einem Gehäuse, welches einen Strömungspfad zwischen einem jeweils mit einem Abschnitt der Infusionsleitung koppelbaren Gehäuseeinlass und Gehäuseauslass definiert, und einer in dem Strömungspfad angeordneten, hydrophilen Filtermembran, welche, insbesondere nach Erliegen einer Strömung/Fluidstrom entlang des Strömungspfads stromauf des Infusionsfilters, eine Luftbarriere ausbildet.

### Hintergrund der Offenbarung

Bei Infusionsbehandlungen wird einem Patienten eine medizinische Flüssigkeit oder eine Infusionslösung über eine Infusionsleitung zugeführt. Die Infusionsleitung ist hierfür mit einem Ende/Endabschnitt an einem Behälter zur Aufbewahrung der Infusionslösung und mit einem anderen Ende/Endabschnitt an einen Patientenzugang oder eine Schnittstelle zu einer weiteren Infusionsleitung angeschlossen.

Um eine Schädigung des Patienten auszuschließen, müssen Fremdkörper, wie z.B. Partikel, in der Infusionslösung gefiltert werden. Ebenso muss sichergestellt werden, dass keine Luft über die Infusionsleitung in das Körperinnere des Patienten gelangt. Wenn der Behälter zur Aufbewahrung der Infusionslösung leerläuft, kann Luft aus dem Behälter in die Infusionsleitung gelangen. Des Weiteren ist ein Lufteintritt in die Infusionsleitung auch durch Restluft aus Verteilern, Ablasshähnen/Absperrventilen oder Injektionsstücken, wie z. B. nadellose oder nadelbasierte Y-Site-Anschlüsse, möglich, vor allem durch ein nicht sachgerechtes bzw. vollständiges Entlüften der Injektionsstücke.

Sobald Fremdkörper und/oder Luft in ein Infusionsset gelangt sind, muss das Infusionsset entweder durch ein neues ersetzt werden oder die Luft muss durch eine geeignete Handhabung des Infusionssets aus der Infusionsleitung entfernt werden, um die Patientenschädigung, insbesondere eine Luftembolie, zu verhindern.

### Stand der Technik

In herkömmlichen Infusionssystemen werden daher üblicherweise Infusionsfilter verwendet. So zeigt beispielsweise EP 1 421 960 A1 einen Infusionsfilter, welcher ein Gehäuse mit einem Einlass und einem Auslass zum Anschließen an die Infusionsleitung aufweist. Um Fremdkörper filtern zu können und einen Lufteintritt in die Infusionsleitung zu verhindern, ist in dem Gehäuse eine hydrophile Filtermembran angeordnet.

Des Weiteren zeigt auch EP 2 500 051 A1 einen Filter zum Einsatz in einem Infusionsset. Innerhalb eines Gehäuses des Filters sind zwei Filterelemente, welche jeweils eine hydrophile Filtermembran aufweisen, angeordnet, um so beim Durchströmen des Filters mit einer Infusionslösung Fremdkörper und Luft aus der Infusionslösung filtern bzw. abscheiden zu können.

Solche bekannten Infusionsfilter benötigen jedoch ein zeitaufwändiges Priming-Verfahren bzw. die zum Primen des Infusionssets benötigte Zeit steigt. Zudem können bei kleiner Filterfläche, d.h. bei Filtern mit geringen Abmessungen/Dimensionen, nur geringe Durchflussmengen/Flussraten realisiert werden und die Infusionsfilter neigen zum Verstopfen/Zusetzen, so dass herkömmliche Infusionsfilter üblicherweise ein sperriges Gehäuse aufweisen.

Auch ist es bekannt, zur Luftabscheidung in herkömmlichen Infusionssystemen Tropfkammern und/oder Luftabscheider einzusetzen. So zeigt beispielsweise DE 299 21 086 U1 ein Infusionsgerät mit einer Tropfkammer, von der eine Infusionsleitung abgeht, und einer in der Tropfkammer am Übergang zu der Infusionsleitung angeordneten hydrophilen Filtermembran, die im befeuchteten Zustand das Eindringen von Luft in die Infusionsleitung blockiert.

Dokumente WO 2020/080889 A1, CA 2 167 932 A1 und US 2013/066272 A1 offenbaren weitere Beispiele für Infusionssets mit Luftfiltern aus dem Stand der Technik.

Herkömmliche Tropfkammern sind dabei im Bereich des Behälters mit der Infusionslösung angeordnet, d.h. am patientenfernen Endabschnitt der Infusionsleitung. Somit bleibt die Gefahr eines Lufteintritts durch Verteiler, Ventile oder andere Verbinder stromab der Tropfkammer weiterhin bestehen. Im Falle einer Mehrfachinfusion, d.h. wenn der Behälter mit der Infusionslösung gewechselt wird, verbleibt so ein großes Restvolumen in der Infusionsleitung.

### Zusammenfassung der Offenbarung

Es ist die Aufgabe und Ziel der Offenbarung, die Nachteile aus dem Stand der Technik zu beheben oder wenigstens zu mindern.

Insbesondere liegt der Offenbarung die Aufgabe zugrunde, einen Infusionsfilter (Luftabscheider, Infusionsvorrichtung) mit hydrophiler Filtermembran zu optimieren, bei dem die Gefahr einer Luftembolie weitgehend ausgeschlossen ist und der imstande ist, bei einer relativ kleinen Baugröße die für Infusionen erforderliche Durchflussmenge/Flussrate zu liefern.

Die Aufgabe wird hinsichtlich eines gattungsgemäßen Infusionsfilters offenbarungsgemäß durch den Gegenstand des Anspruchs 1 gelöst. Die Offenbarung beruht also auf der Erkenntnis, einer Turbulenzbildung in der Strömung entgegenzuwirken bzw. eine Turbulenzbildung in der Strömung zu verhindern.

Demzufolge ist der Infusionsfilter offenbarungsgemäß konfiguriert/dafür angepasst, dass das Gehäuse so ausgebildet ist, dass die Strömung entlang des gesamten Strömungspfads turbulenzfrei (laminar) ist. Insbesondere weist das Gehäuse stromauf der Filtermembran ein erstes Strömungsleitelement in Form eines Krümmers für eine erste Umlenkung einer Strömung entlang des Strömungspfads quer, insbesondere senkrecht, zur Hauptströmungsrichtung und ein unmittelbar darauf folgendes zweites Strömungsleitelement in Form einer schräg zur Hauptströmungsrichtung ausgerichteten Prallfläche für eine zweite Umlenkung der Strömung zurück in die Hauptströmungsrichtung aufweist, um so ein Anströmen der Filtermembran ausschließlich an deren stromaufwärtigen Membranseite zu gewährleisten. D.h. das Gehäuse stromauf der Filtermembran weist ein erstes Strömungsleitelement in Form eines Krümmers für eine erste Umlenkung einer Strömung entlang des Strömungspfads quer, insbesondere senkrecht, zu einer Hauptströmungsrichtung und ein zweites Strömungsleitelement in Form einer schrägen Prallfläche (Schräge /Rampe/Fase) für eine zweite Umlenkung der Strömung in die Hauptströmungsrichtung auf. So kann die Strömung innerhalb des Gehäuses sanft, d.h. ohne Ablösungen zu provozieren bzw. Toträume/Totwassergebiete/Ablöseblasen zu verursachen, umgelenkt und eine optimale Anströmung der Filtermembran gewährleistet werden.

Vorteilhafte Ausführungsformen sind in den Unteransprüchen beansprucht und werden nachfolgend erläutert.

In einer vorteilhaften Ausgestaltung kann die, vorzugsweise tellerförmige, Filtermembran so in dem Gehäuse angeordnet sein, dass sie sich im Wesentlichen in der bzw. entlang der Hauptströmungsrichtung erstreckt. D.h. die Filtermembran kann so in dem Gehäuse angeordnet sein, dass eine Normale auf einer der Stirnflächen der Filtermembran senkrecht zu der Hauptströmungsrichtung ist. Anders ausgedrückt kann die Filtermembran so in dem Gehäuse angeordnet sein, dass die Strömung durch die Filtermembran im Wesentlichen senkrecht zur Hauptströmungsrichtung ist.

Gemäß einer offenbarungsgemäßen Weiterbildung kann das Gehäuse stromab der Filtermembran ein drittes Strömungsleitelement in Form eines Krümmers, welches die im Wesentlichen senkrecht durch die Filtermembran strömende Strömung in die Hauptströmungsrichtung umlenkt, aufweisen. Somit kann auch stromab der Filtermembran verhindert werden, dass sich Totwassergebiete bilden, in welchen sich residuale Luftblasen sammeln können.

Mit anderen Worten kann die Strömung entlang des Strömungspfads mittels Strömungsleitelementen, vorzugsweise in Form von Schrägen und radialen/gerundeten Umlenkungen, geführt sein. Anders ausgedrückt können Strömungsleitelemente zur Führung entlang des Strömungspfads als Schräge/schräge Prallfläche/Rampe/Fase oder Radien/gerundete Umlenkungen ausgeführt sein, d.h. die Strömungsführung innerhalb des Infusionsfilters kann ohne Ecken oder Hinterschnitte erfolgen, so dass sich keine Totwassergebiete, an welchen Luftblasen festsetzen oder entstehen können, ausbilden können. Insbesondere können die Umlenkbereiche im Bereich des Gehäuseeinlasses und des Gehäuseauslasses in der Form eines Krümmers (einer geviertelten Kugel bzw. eines Viertel-Torus) ausgeführt sein, was eine besonders sanfte Umlenkung der Strömung, auch um mehr als 45°, insbesondere 90°, erlaubt. Ein Radius des Krümmers kann dabei vorzugsweise zwischen 1mm und 2mm, insbesondere 1,5mm betragen. Des Weiteren kann an einem Einströmbereich, welcher sich zu einem in dem Gehäuse ausgebildeten Hohlraum (Kavität) zur Aufnahme der Filtermembran öffnet, die schräge Prallfläche ausgebildet sein, welche ähnlich den Umlenkbereichen eine sanfte Umlenkung und eine optimierte Anströmung der Filtermembran ermöglicht. Ein Winkel der Schräge kann dabei beliebig an den Einströmbereich angepasst sein. Vorzugsweise kann der Winkel zwischen 5° und 35°, insbesondere zwischen 10° und 20°, besonders bevorzugt um 15°, betragen.

Gemäß einer vorteilhaften Ausgestaltung können der Gehäuseeinlass und der Gehäuseauslass einen Aufnahmeabschnitt/Aufnahmebereich zur Aufnahme des jeweiligen Infusionsleitungsabschnitts aufweisen, und die Aufnahmeabschnitte so an Dimensionen der Infusionsleitungsabschnitte angepasst sein, dass im gekoppelten Zustand Innendurchmesser der Abschnitte der Infusionsleitung oberflächenbündig mit den Aufnahmeabschnitten abschließen. Besonders bevorzugt können die Aufnahmeabschnitte als Anschlag für die Infusionsleitung jeweils eine umlaufende Stufe (Absatz) aufweisen, deren Höhe im Wesentlichen einer Wandstärke/Dicke der Infusionsleitung entspricht, d.h. wenn die Infusionsleitung in den Infusionsfilter eingeschoben/eingelegt/eingeführt ist, bilden sich keine Spalte oder Stufen an der Schnittstelle/Übergang zwischen der Infusionsleitung und dem Infusionsfilter aus, was eine Ausbildung eines Spalts/Absatzes an dieser Schnittstelle verhindert, wodurch wiederum ein Festsetzen von residualen Luftblasen vermieden werden kann.

Ferner kann es offenbarungsgemäß besonders von Vorteil sein, wenn das Gehäuse ein Gehäuseunterteil und einen Gehäusedeckel aufweist, welche form- und/oder kraft- und/oder stoffschlüssig miteinander verbindbar sind und dabei eine Kavität im Gehäuseinneren ausbilden.

Zudem kann es zweckmäßig sein, wenn der offenbarungsgemäße Infusionsfilter einen in dem Gehäuse angeordneten, luftdurchlässigen Belüftungsfilter aufweist, welcher über eine in dem Gehäuse ausgebildete Öffnung mit einer Gehäuseaußenseite in Verbindung steht. So können residuale Luftblasen, welche sich, insbesondere nach Erliegen der Strömung stromauf des Infusionsfilters, innerhalb des Gehäuses sammeln/festsetzen, ausströmen/abfließen. Gleichzeitig verhindert der Belüftungsfilter ein Eintreten von Fremdkörpern.

**In** einer vorteilhaften Weiterbildung kann das Gehäuse eine Stützstruktur zur Aufnahme der Filtermembran aufweisen. Dabei kann die Stützstruktur einen der Geometrie der Filtermembran folgenden, geschlossenen, vorzugsweise kreisrunden, Steg und innerhalb dieses Stegs ausgebildete Unterstützungselemente, insbesondere Rippen und Noppen (Unterstützungspunkte), aufweisen. Die Stützstruktur gewährleistet zum einen eine sichere Aufnahme der Filtermembran und verhindert zum anderen die Ausbildung möglicher Turbulenzen oder residualer Luftblasen. Dabei kann es besonders zweckmäßig sein, wenn die Rippen in Hauptströmungsrichtung ausrichtend stromab zur Filtermembran unmittelbar dieser nachfolgend angeordnet sind und die Noppen stromab zu den Rippen sowie vorzugsweise bezüglich der Hauptströmungsrichtung querversetzt zu den Rippen platziert sind.

Weiterhin ist es bevorzugt, wenn die Filtermembran einen Blasendruck zwischen 0,2 und 0,45 bar und/oder eine Fläche unter 2,0 cm², vorzugsweise unter 1,7 cm², hat, was eine hohe Flussrate bei ausreichender Filter-/Barrierenwirkung ermöglicht.

Zudem kann es zweckmäßig sein, wenn die Filtermembran so ausgebildet ist, dass die hydraulische Permeabilität für Wasser (Wasserflussrate) bei einem Druck von 0,1 bar mindestens 120 ml/min, vorzugsweise mindestens 140 ml/min, beträgt. Eine solche Filtermembran erlaubt den Einsatz des offenbarungsgemäßen Infusionsfilters in standardisierten Infusionsleitungen und für alle Infusionsbehandlungen, da die Wasserflussrate den normativ geforderten Wert von 1000ml/10min (ISO 8536-4) übersteigt.

Gemäß einer bevorzugten Ausgestaltung kann die Filtermembran aus einer Vielzahl an parallelen Röhrchen gebildet sein. Alternativ kann die Filtermembran auch aus einem Block mit Spalten, aus Lamellen oder einem offenen porösen Material, insbesondere Membran, Filz oder Schaum, aufgebaut sein. In einer solchen Filtermembran entstehende/vorherrschende Kapillarkräfte stellen die Funktion/Wirkung der Filtermembran als Luftbarriere aufgrund eines kapillaren Flussstopps sicher.

Des Weiteren betrifft die Offenbarung ein Infusionsset mit einer Infusionsleitung, deren erster Endabschnitt einen Anschluss für einen Behälter mit einer Infusionslösung und deren zweiter Endabschnitt einen Anschluss für einen Patientenzugang oder eine weitere Infusionsleitung aufweist, und einem in der Infusionsleitung angeordneten/zwischengeschalteten offenbarungsgemäßen Infusionsfilter. Dabei kann der Infusionsfilter in Strömungsrichtung der Infusionslösung von dem ersten Endabschnitt zu dem zweiten Endabschnitt unmittelbar vor dem zweiten Endabschnitt angeordnet sein oder auch direkt in dem Anschluss für den Patientenzugang integriert sein, d.h. das in der Infusionsleitung verbleibende Restvolumen ist gering und eine mögliche Ausbildung/Festsetzung residualer Luftblasen in möglicherweise stromab (zwischen Infusionsfilter und Anschluss für Patientenzugang) angeordneten, weiteren Komponenten, wie z.B. Absperrventilen o.ä., kann vermieden werden. Zudem kann es bei einem offenbarungsgemäßen Infusionsset zweckmäßig sein, wenn eine in der Infusionsleitung angeordnete Tropfkammer und/oder eine an der Infusionsleitung angeordnete Klemmvorrichtung vorgesehen ist.

Anders ausgedrückt betrifft die Offenbarung einen kostengünstigen Infusionsfilter für ein Infusionsset, welcher es ermöglicht, eine Luftstopp-Funktion, d.h. eine Luftbarriere, von einer Tropfkammer an das Ende der Infusionsleitung zu verlegen, um das Restvolumen zu reduzieren und Luft von anderen Quellen, wie z.B. Y-Anschlussstücken, Absperrventilen oder Entlüftung, zu beseitigen. Dabei ist die Offenbarung gekennzeichnet dadurch, dass der Infusionsfilter Luft aus einer Infusionsleitung eliminiert/abscheidet und die Strömung automatisch stoppt. Die Luftabscheidung findet hierbei nahe dem Ende der Infusionsleitung im Nahbereich des Patienten statt, was zu einem niedrigen/geringen Restvolumen und einer effizienten Luftabscheidung führt. Zudem ist der Infusionsfilter schnell und einfach zu primen, was eine spezielle Priming-Prozedur oder zusätzliche Schritte bei der Handhabung durch einen Verwender obsolet macht. Des Weiteren sieht der Infusionsfilter auch eine hohe Durchflussmenge bei minimierter Größe und sehr geringem Restvolumen sowie eine effektive Rückhaltung von Partikeln mit einer Größe von wenigstens 3 µm (Partikelgröße ≥ 3 µm). Eine Abwägung der Spezifikationen der hydrophilen Fluidfiltermembran und der spezifizierten Fläche der hydrophilen Fluidfiltermembran sorgt für eine hohe Flussrate in Kombination mit einer sehr kleinen und nicht-sperrigen Größe des Infusionsfilters. Dafür hat die hydrophile Fluidfiltermembran einen Blasendruck zwischen 200 und 450 mbar und die Fläche der hydrophilen Fluidfiltermembran liegt unter 2,0 cm², insbesondere unter 1,7 cm². Infusionssets mit einem integrierten Infusionsfilter haben demnach bei einer Druckdifferenz von 0,1 bar eine Wasserflussrate von mindestens 120 ml/min, vorzugsweise 140 ml/min. Das Gehäuse des Infusionsfilters hat ein Design, das in jeder Position des Infusionsfilters schnelles Primen erlaubt, ohne eine spezielle Priming-Prozedur oder zusätzliche Schritte bei der Handhabung durch den Verwender. Die Priming-Zeit des Infusionsfilters liegt bei unter 15s, vorzugsweise unter 10s, besonders bevorzugt bei unter 5s. Das Restvolumen des Infusionsfilters ist unterhalb 0,2 ml, vorzugsweise unterhalb 0,16 ml. Hierbei ist der Infusionsfilter primär als ein Set aus hydrophilen Kapillaren, und nicht als Luftfilter zur Keimrückhaltung, designt. Das Set aus hydrophilen Kapillaren soll Luft und Partikel mit einer Partikelgröße von mindestens 3 µm zurückhalten. Somit ermöglicht die Offenbarung schnelles Primen der Infusionsleitung und des Infusionsfilters in jeder Orientierung ohne eine spezielle Priming-Prozedur, was eine Zeiteinsparung für den Verwender während der Vorbereitungen bedeutet. Die geringen Dimensionen des Gehäuses des Infusionsfilters wiederum führen zu einer verringerten Krafteinwirkung auf die Infusionsleitung, helfen bei der Vorbeugung gegen Stecken- oder Hängenbleiben des Infusionsfilters in oder an anderen Infusionsleitungen oder anderen Objekten und führen zu einem niedrigen Restvolumen, was in einem minimalen Verlust der Medikation am Ende der Infusion resultiert. Die hohe Wasserflussrate gewährleistet einen schnellen Volumenaustausch bei Notfallsituationen. Aufgrund der effizienten Partikelrückhaltung kann auch die Partikellast auf den Patienten während der Infusionsbehandlung reduziert werden. Darüber hinaus hilft die effiziente Luftabscheidung in (unmittelbarer) Nähe zum Patienten, Lufteintritt in den Patienten während der Infusionsbehandlung vorzubeugen. Hierfür hat das Gehäuse des Infusionsfilters einen optimierten Strömungspfad ohne Toträume, um ein schnelles und Luftblasen-freies Primen zu erreichen. Aufgrund diesem Design funktioniert das Luftblasen-freie Primen unabhängig von der Orientierung des Infusionsfilters und benötigt keine spezielle Priming-Prozedur oder zusätzliche Handhabung durch den Verwender. In einer Variante kann der Infusionsfilter die hydrophile Fluidfiltermembran sein, welche einen Blasendruck zwischen 200 und 450 mbar, eine Luftstromflussrate von mehr als 5,5 LSL (ft3/ft2/min), vorzugsweise 6,5 LSL, bei einem Druck von 125 Pa im unbenetzten (trockenen) Zustand, eine Wasserflussrate von mehr als 1200, insbesondere 1400, ml/cm²/min bei einem Druck von 10 psi und eine Fläche von weniger als 2,0 cm², insbesondere weniger als 1,7 cm², hat.

In anderen Worten ausgedrückt, sind die Baugröße bzw. die inneren Kavitäten des Infusionsfilters so klein wie möglich gehalten. Es gibt keine scharfkantigen 90° Übergänge. Alle Übergänge können als Radien oder Schrägen ausgeführt sein. Zusätzlich kann eine Schräge im Gehäusedeckel dazu beitragen, den Fluidstrom entsprechend zu lenken und ohne Turbulenzen hin zur Fluidfiltermembran zu führen. In dem Gehäuse kann der Bereich zwischen Einlassöffnung und Rand der Kavität komplett mit Kunststoff gefüllt sein, um hier die Ausbildung eines Totraums zu verhindern. Weiterhin können Unterstützungen für den Flüssigkeitsfilter mögliche Turbulenzen und residuale Luftblasen beim Primen des Filters verhindern. Bei dem offenbarungsgemäßen Infusionsfilter können für diese Unterstützungen eine Kombination aus Rippen und einzelnen Unterstützungspunkten/Noppen gewählt werden. Herkömmliche Infusionsfilter haben in der Regel nur Rippen, zwischen denen noch residuale Luft anhaften kann. Die Dimensionen und Abmessungen des Gehäuseeinlasses und des Gehäuseauslasses können an Dimensionen der Infusionsleitung (Schlauchdimensionen) angepasst werden, so dass keine Absätze/Stufen bzw. keine Spalte zwischen Infusionsleitung/Schlauch und Schlauchansatz vorliegen, was verhindert, dass sich hier residuale Luftblasen festsetzen können. Die Umlenkung zum Gehäuse kann zudem kugelförmig ausgeführt sein. Die Schräge im Gehäusedeckel kann den Fluidstrom ohne Turbolenzen oder "Lufteinschlüsse" in Richtung Fluidfiltermembran lenken. Der Winkel der Schräge kann dabei an den Abstand zwischen dem Rand der Kavität und der Einlassöffnung angepasst werden und kann zwischen 10° und 20° liegen und insbesondere 15° sein. In dem Gehäuseunterteil kann der entsprechende Bereich nicht als Schräge ausgeführt, sondern komplett mit Kunststoff gefüllt sein. Diese Kombination aus der Schräge im Gehäusedeckel und des "gefüllten" Bereiches im Gehäuseunterteil kann den Fluidstom entsprechend lenken und dadurch gewährleisen, dass die Fluidfiltermembran beim Primen optimal angeströmt und benetzt werden kann. Durch die optimale Anströmung beim Benetzen kann wiederum der Einschluss von Luftblasen nach der Filtermembran während der Befüllung des Infusionsfilters vermieden werden.

Mit noch anderen Worten, betrifft die Offenbarung einen Infusionsfilter mit einer hohen (Wasser-)Flussrate/Durchflussmenge von über 120 ml/min, vorzugsweise von über 140 ml/min, bei einem Druck von 0,1 bar in Kombination mit einer geringen Größe des Infusionsfilters bzw. einer kleinen Fläche der Fluidfiltermembran. Der offenbarungsgemäße Infusionsfilter ist darüber hinaus auch geeignet für Infusionslösungen mit einer höheren Viskosität, wie z.B. Glukoselösung oder Lipid-Emulsion. Um eine blasenfreie Füllung/Priming des Infusionsfilters zu erreichen, sind keine spezielle Priming-Prozedur oder keine zusätzliche Handling-Schritte durch den Verwendet nötig. Die blasenfreie Füllung des Infusionsfilters funktioniert unabhängig von seiner Position/Orientierung im Raum. Der offenbarungsgemäße Infusionsfilter hat einen optimierten Strömungspfad, wobei im Inneren des zusammengebauten Infusionsfilters keine Totwassergebiete, in welchen Luftblasen während der Füllung des Infusionsfilters festhängen, vorhanden sind. Zudem sind die Position der Einlass- und Auslassöffnung in der Kavität gewählt, um eine optimale Strömung der Infusionslösung zu der Filtermembran sicherzustellen, ohne einen Einschluss von Luftblasen während der Füllung.

Weitere Vorteile sind, dass ein Umstecken des Infusionsbesteckes von einem leergelaufenen Infusionsbehälter in einen neuen, vollen Infusionsbehälter ohne Manipulation des Anwenders möglich ist. Des Weiteren wird ein lageunabhängiges Entlüften ermöglicht. Bei der Bolusgabe über nicht sachgerecht entlüftete Bolusinjektionsports, Ventile oder Dreiwegehähne wird die Luft eliminiert und mögliche Partikel während dem Zuspritzen werden zurückgehalten. Dieses bietet sowohl gesteigerten Anwenderkomfort als auch erhöhte Patientensicherheit.

### Kurzbeschreibung der Figuren

Die Offenbarung wird nachfolgend anhand bevorzugter Ausführungsbeispiele mit Hilfe von Figuren näher erläutert. Es zeigen:
- Fig. 1: eine Perspektivansicht eines Infusionsfilters gemäß einem bevorzugten Ausführungsbeispiel,
- Fig. 2: eine Explosionsdarstellung des Infusionsfilters gemäß dem bevorzugten Ausführungsbeispiel,
- Fig. 3: eine Perspektivansicht eines Gehäuseunterteils des Infusionsfilters gemäß dem bevorzugten Ausführungsbeispiel,
- Fig. 4: eine Längsschnittdarstellung des Gehäuseunterteils des Infusionsfilters gemäß dem bevorzugten Ausführungsbeispiel,
- Fig. 5: eine Perspektivansicht eines Gehäusedeckels des Infusionsfilters gemäß dem bevorzugten Ausführungsbeispiel,
- Fig. 6: eine Ansicht von unten des Gehäusedeckels des Infusionsfilters gemäß dem bevorzugten Ausführungsbeispiel,
- Fig. 7a und 7b: schematische Darstellungen der Wirkweise einer hydrophilen Filtermembran des Infusionsfilters gemäß dem bevorzugten Ausführungsbeispiel,
- Fig. 8: eine Längsschnittdarstellung des mit einer Infusionsleitung verbundenen Infusionsfilters gemäß dem bevorzugten Ausführungsbeispiel, und
- Fig. 9: eine Perspektivansicht eines Infusionssets mit dem Infusionsfilter gemäß dem bevorzugten Ausführungsbeispiel.

### Detaillierte Beschreibung eines bevorzugten Ausführungsbeispiels

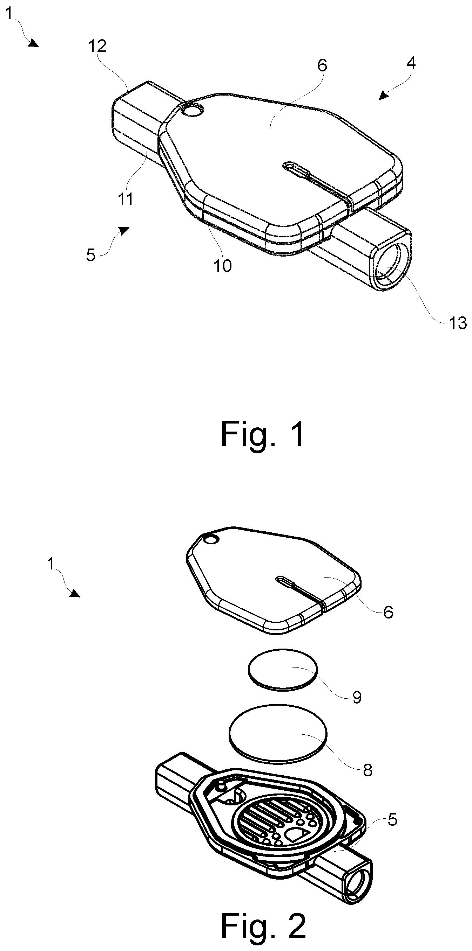

Fig. 1 zeigt eine perspektivische Ansicht und Fig. 2 eine Explosionsdarstellung eines Infusionsfilters/Luftabscheiders 1 gemäß einem bevorzugten Ausführungsbeispiel für ein nachstehend näher beschriebenes Infusionsset 2, d.h. der Infusionsfilter 1 ist eingerichtet, an eine, eine Infusionslösung führende Infusionsleitung 3 angeschlossen bzw. in die Infusionsleitung 3 zwischengeschalten zu werden.

Wie in Fig. 1 gezeigt, weist der Infusionsfilter 1 ein Gehäuse 4 mit einem Gehäuseunterteil 5 und einem form- und/oder kraftschlüssig mit dem Gehäuseunterteil 5 verbundenen Gehäusedeckel 6 auf.

Im Inneren des Gehäuses 4, d.h. im Bereich einer zwischen dem Gehäuseunterteil 5 und dem Gehäusedeckel 6 ausgebildeten Kavität (Hohlraum) 7, sind, wie in Fig. 2 zu erkennen, eine hydrophile (Fluid-)Filtermembran 8 und ein Entlüftungs-/Belüftungsfilter 9 angeordnet. Wie nachstehend näher beschrieben, dient die Filtermembran 8 dabei als Luftbarriere und der Belüftungsfilter 9 ermöglicht den Transport von Luft, welche sich im Bereich der Kavität 7 sammelt, auf eine Außenseite des Gehäuses 4. Die Filtermembran 8 teilt das Gehäuse 4 folglich in einen stromaufwärtigen Gehäuseabschnitt 4a und einen stromabwärtigen Gehäuseabschnitt 4b.

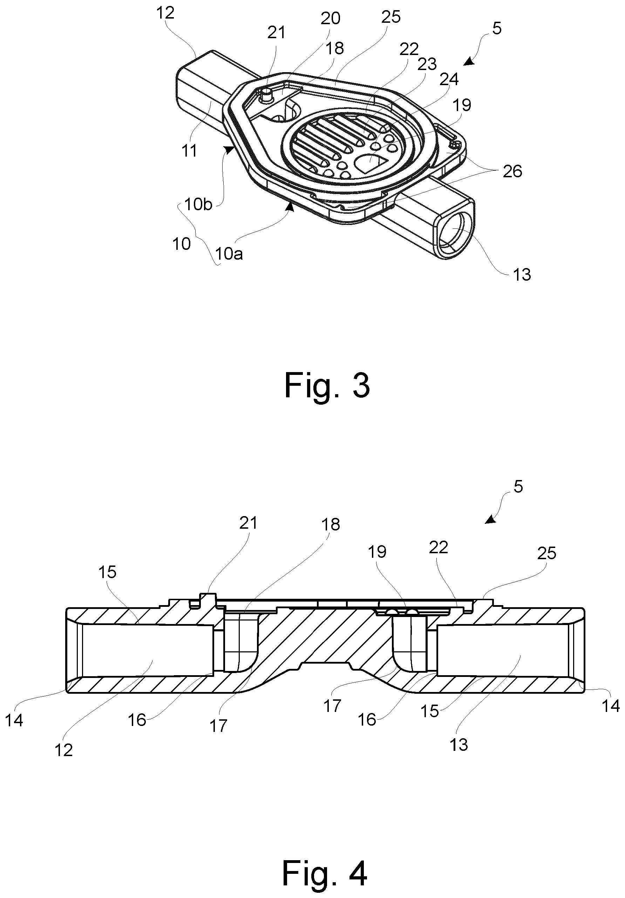

Fig. 3 zeigt eine perspektivische Ansicht des Gehäuseunterteils 5 des Infusionsfilters 1 gemäß dem bevorzugten Ausführungsbeispiel. Das Gehäuseunterteil 5 hat einen Hauptkörper 10, welcher in einer Draufsicht in Form eines Wappenschilds ausgestaltet ist, d.h. der Hauptkörper 10 hat eine symmetrische Polygonform mit einem breiten ersten Abschnitt 10a und einem zulaufenden zweiten Abschnitt 10b. Ferner hat das Gehäuseunterteil 5 einen auf einer Unterseite des Hauptkörpers 10 angeordneten Balkenkörper 11, welcher sich in Längsrichtung über den ersten und den zweiten Abschnitt 10a, 10b des Hauptkörpers 10 hinaus erstreckt. Anders ausgedrückt, weist das Gehäuseunterteil 5 den Hauptkörper 10 und den Balkenkörper 11 auf, wobei der Balkenkörper 11 in seiner Erstreckungsrichtung länger als der Hauptkörper 10 und quer zu seiner Erstreckungsrichtung schmäler als der Hauptkörper 10 ist.

Des Weiteren hat der Balkenkörper 11, wie in Fig. 4 gezeigt, an seinen beiden Stirnflächen jeweils eine Öffnung, welche jeweils mit der zwischen dem Gehäuseunterteil 5 und dem Gehäusedeckel 6 ausgebildeten Kavität 7 in Fluidverbindung steht. Der Balkenkörper 11 bildet demzufolge einen Gehäuseeinlass 12 und einen Gehäuseauslass 13, wobei der Gehäuseeinlass 12 bezüglich des Hauptkörpers 10 auf der Seite des zweiten Abschnitts 10b und der Gehäuseauslass 13 auf der Seite des ersten Abschnitts 10a ausgebildet ist.

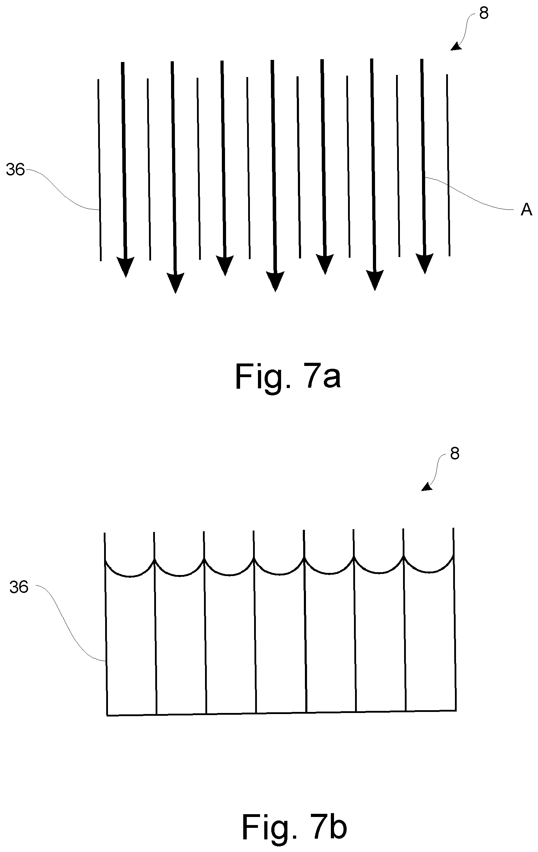

Wie vorstehend erwähnt, ist der Infusionsfilter 1 gemäß dem bevorzugten Ausführungsbeispiel eingerichtet, um mit der Infusionsleitung 3 fluidisch verbunden zu werden. D.h. der Gehäuseeinlass 12 kann mit einem Stromauf-Abschnitt der Infusionsleitung 3 verbunden werden, wohingegen der Gehäuseauslass 13 mit einem Stromab-Abschnitt der Infusionsleitung 3 gekoppelt werden kann. Hierfür werden der Stromauf-Abschnitt und der Stromab-Abschnitt in den Gehäuseeinlass 12 bzw. den Gehäuseauslass 13 geschoben. Für eine leichtere Handhabung beim Einführen/Einschieben der Infusionsleitung 3 weisen der Gehäuseeinlass 12 und der Gehäuseauslass 13 jeweils eine als Fase/Schräge ausgebildete Einführstruktur 14 auf. An diese Einführstruktur 14 schließt sich in Einführrichtung jeweils ein Aufnahmebereich 15 für die jeweiligen Abschnitte der Infusionsleitung 3 an. An dem der Einführstruktur 14 abgewandten Endbereich der Aufnahmebereiche 15 ist jeweils eine umlaufende Stufe 16 als Anschlag für die Abschnitte der Infusionsleitung 3 ausgebildet. Wie in Fig. 7 gezeigt, entspricht eine Höhe der Stufe 16 dabei im Wesentlichen einer Wandstärke der Infusionsleitung 3, d.h. die Aufnahmebereiche 15 sind so an die Infusionsleitung 3 angepasst, dass sich bei eingeschobener Infusionsleitung 3 keine Stufen/Absätze ergeben und so mögliche Verwirbelungen, welche zur Ausbildung von Luftblasen führen können, durch die Vermeidung von Absätzen/Stufen entlang des Strömungspfads verhindert werden. Anders ausgedrückt sind Dimensionen der Aufnahmebereiche 15 des Gehäuseeinlasses 12 und des Gehäuseauslasses 13 so an Dimensionen der Infusionsleitung 3 angepasst, dass keine Absätze oder Spalten zwischen Infusionsleitung 3 und Aufnahmebereich 15 vorliegen. In noch anderen Worten ausgedrückt, sind die Aufnahmebereiche 15 des Gehäuseeinlasses 12 und des Gehäuseauslasses 13 so an die Dimensionen der Infusionsleitung 3 angepasst, dass im eingeführten Zustand, d.h. wenn die Infusionsleitung 3 in den Infusionsfilter 1 eingeschoben wurde, Innendurchmesser der Infusionsleitung 3 oberflächenbündig mit den Stufen 16 abschließen. Dies verhindert, dass sich hier residuale Luftblasen festsetzen können.

An die Stufe 16 schließt sich jeweils, d.h. auf der Seite des Gehäuseeinlasses 12 und des Gehäuseauslasses 13, ein Umlenkbereich/Krümmer 17, über welchen eine Strömung der Infusionslösung um ca. 90° umgelenkt wird, an. Wie in Fig. 4 gezeigt, sind die Umlenkbereiche 17 gerundet ausgeführt bzw. haben die Form einer geviertelten Kugel, deren eine offene Schnittfläche hin zu dem Gehäuseeinlass 12 bzw. zu dem Gehäuseauslass 13 ausgerichtet ist und deren andere offene Schnittfläche unter Ausbildung eines rechten Winkels zu der einen offenen Schnittfläche angeordnet ist. Mit noch anderen Worten ausgedrückt, ist die Umlenkung in dem Gehäuse 4 kugelförmig ausgeführt, so dass die Strömung umgelenkt werden kann, ohne dass sich Turbulenzen oder Totwassergebiete bilden.

Wie in Fig. 4 zu erkennen, ist der Infusionsfilter 1 gemäß dem bevorzugten Ausführungsbeispiel so gestaltet, dass sich die vorstehend beschriebenen Elemente, d.h. die Einführstruktur 14, der Aufnahmebereich 15, die Stufe 16 und der Umlenkbereich 17, für den Gehäuseeinlass 12 und den Gehäuseauslass 13 gleichen. D.h. der Gehäuseeinlass 12 ist hinsichtlich einer Mittenebene des Infusionsfilters 1 im Wesentlichen symmetrisch zu dem Gehäuseauslass 13 ausgebildet.

Auf der Seite des Gehäuseeinlasses 12 schließt sich an den Umlenkbereich 17 eine sich zur Kavität 7 hin öffnende Einlassöffnung 18 an, wohingegen auf der Seite des Gehäuseauslasses 13 eine zur Kavität 7 hin offene Auslassöffnung 19 ausgebildet ist. Sowohl die Einlassöffnung 18 als auch die Auslassöffnung 19 sind in einer Draufsicht auf das Gehäuseunterteil 5 in Form einer halben Ellipse ausgestaltet, wobei die Radien der halben Ellipsen zueinander ausgerichtet sind.

Wie in Fig. 3 zu erkennen, ist auf einer der Auslassöffnung 19 abgewandten Seite der Einlassöffnung 18 ein Montageabschnitt 20 mit einem vorragenden Montagezapfen 21 ausgebildet. Auf einer der Auslassöffnung 19 zugewandten Seite der Einlassöffnung 18 ist ein kreisrunder Steg 22 angeordnet, innerhalb dessen die Auslassöffnung 19 sowie in Erstreckungsrichtung des Balkenkörpers 11 ausgerichtete Rippen 23 und einzelne Noppen 24 ausgebildet sind. Wie nachstehend näher beschrieben, dienen der Steg 22, die Rippen 23 und die Noppen 24 als eine Stützstruktur für die in dem Gehäuseunterteil 5 aufgenommene Filtermembran 8. Diese Stützstruktur für die Filtermembran 8 kann mögliche Turbulenzen und residuale Luftblasen beim Primen des Infusionsfilters 1 verhindern.

In einem Randbereich des Hauptkörpers 10 ist ein weiterer Steg 25 ausgebildet, welcher im Bereich des zweiten Abschnitts 10b der Form des Hauptkörpers 10 folgt und im Bereich des ersten Abschnitts 10a halbkreisförmig verläuft. Anders ausgedrückt, ist der Steg 25 im Bereich des zweiten Abschnitts 10b vom Rand des Hauptkörpers 10 so nach innen abgesetzt, dass sich eine Stufe mit konstanter Breite ergibt. Im Bereich des ersten Abschnitts 10a beschreibt der Steg 25 eine Halbkreisform, so dass Schulterbereiche 26 des Hauptkörpers 10 freigelegt werden, welche, wie nachstehend näher beschrieben wird, zur Aufnahme des Gehäusedeckels 6 dienen.

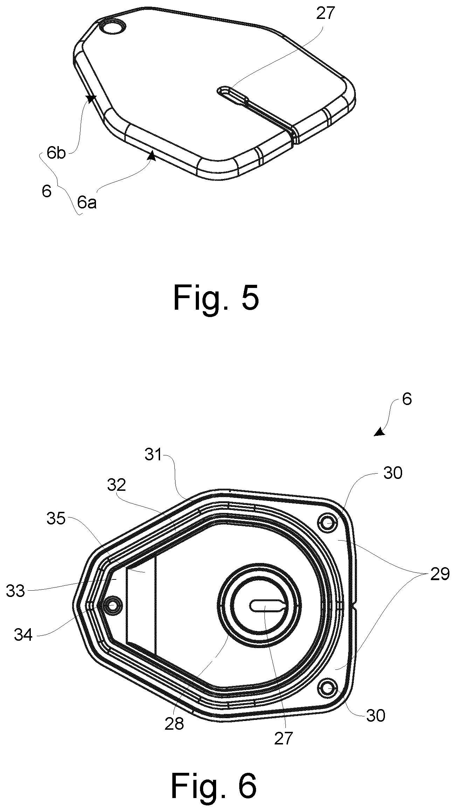

Fig. 5 und 6 zeigen Ansichten des Gehäusedeckels 6 des Infusionsfilters 1 gemäß dem bevorzugten Ausführungsbeispiel. Der Gehäusedeckel 6 hat, ähnlich dem Hauptkörper 10 des Gehäuseunterteils 5, eine Wappenschildform mit einem ersten, breiten Abschnitt 6a und einem zweiten, zulaufenden Abschnitt 6b. Im Bereich des ersten Abschnitts 6a ist mittig eine durchgehende Öffnung in Form eines Langlochs (Belüftungsöffnung) 27 ausgebildet. Wie in Fig. 6 zu erkennen, ist auf einer Innenseite des Gehäusedeckels 6, welche im montierten Zustand des Infusionsfilters 1 der Kavität 7 zugewandt ist, um das Langloch 27 ein kreisrunder Steg 28 ausgeformt, welcher, wie nachstehend näher beschrieben wird, zur Aufnahme des Belüftungsfilters 9 dient. In Schulterbereichen 29 des ersten Abschnitts 6a des Gehäusedeckels 6 sind jeweils Montagezapfen 30 angeordnet, welche bei der Montage des Infusionsfilters 1 gemäß dem bevorzugten Ausführungsbeispiel mit den Schulterbereichen 26 des Hauptkörpers 10 zusammenwirken.

In einem Randbereich des Gehäusedeckels 6 ist ein erster, im Querschnitt dreieckiger Steg 31 ausgeformt, welcher im Wesentlichen der Form des Stegs 25 des Hauptkörpers 10 folgt und bei der Montage des Infusionsfilters 1 auf diesem aufliegt bzw. gegen diesen gepresst/gedrückt wird und so eine Dichtung darstellt. In anderen Worten, dichten der Steg 25 des Hauptkörpers 10 und der Steg 31 des Gehäusedeckels 6 das Gehäuseunterteil 5 und den Gehäusedeckel 6 gegeneinander ab.

Innerhalb des ersten Stegs 31 ist ein umlaufender und der Form des ersten Stegs 31 folgender, zweiter Steg 32 ausgebildet. Im Bereich des zweiten Abschnitts 6b, d.h. an dem zulaufenden Endabschnitt des Gehäusedeckels 6, ist ein Montagebereich 33 mit einer Montageöffnung 34 in Form einer Sacklochbohrung angeordnet, so dass der Montagezapfen 21 bei Montage des Infusionsfilters 1 gemäß dem bevorzugten Ausführungsbeispiel in die Montageöffnung 34 greift. Sowohl in dem Gehäuseunterteil 5 als auch in dem Gehäusedeckel 6 ist der Montagebereich 20 bzw. 33 zwischen Einlassöffnung 18 und Steg 25 komplett mit Kunststoff gefüllt, d.h. aus Vollmaterial gebildet. Somit kann hier die Ausbildung eines Totwassergebiets verhindert werden.

Auf einer dem Langloch 27 zugewandten Seite des Montagebereichs 33 ist eine Rampe/Fase/Schräge 35 ausgebildet, welche, wie nachstehend näher beschrieben wird, eine kontrollierte, turbulenzfreie Umlenkung der Strömung durch den Infusionsfilter 1 gewährleistet, d.h. die Schräge 35 im Gehäusedeckel 6 trägt dazu bei, die Strömung entsprechend zu lenken und ohne Turbulenzen in Richtung hin zur Filtermembran 8 zu führen. In dem Infusionsfilter 1 gemäß dem bevorzugten Ausführungsbeispiel beträgt der Winkel der Schräge 35 in etwa 15°. Selbstverständlich kann die Schräge 35 aber auch einen anderen Winkel annehmen. Insbesondere ist der Winkel der Schräge 35 an den Abstand zwischen dem Steg 25 und der Einlassöffnung 18 angepasst.

Der Gehäusedeckel 6 kann auf der Außenseite mit einer oder mehreren Belüftungsnuten ("venting groove") ausgestattet sein. Die Belüftungsnuten verhindern ein Verschließen der Belüftungsöffnung 27 (durch z.B. Fixation des Luftabscheiders 1 am Patienten oder durch die Lage des Patienten). Die Belüftungsnuten führen von der Belüftungsöffnung 27 zu den Außenkanten des Gehäusedeckels 6. Um Druckstellen auf der Haut zu verhindern, kann der Gehäusedeckel 6 ohne erhabene Rippen oder sonstige erhabene Bereiche ausgeführt sein.

Die Fign. 7a und 7b verdeutlichen schematisch das Wirkprinzip der Filtermembran 8. In dem bevorzugten Ausführungsbeispiel, ist die Filtermembran 8 als ein Set/Satz von hydrophilen Kapillaren, welche als eine Vielzahl an zusammengepackten, parallelen Röhrchen 36 angesehen werden können, aufgebaut. Wenn nun eine Flüssigkeit durch die Filtermembran 8 strömt, strömt diese durch die parallelen Röhrchen 36 (Pfeile A in Fig. 7a). Wie vorstehend erwähnt, strömt in dem Infusionsset 2 als Flüssigkeit die Infusionslösung aufgrund der Schwerkraft. D.h. das Gewicht der Flüssigkeit stellt den zum Strömen benötigten Druck bereit.

Wenn nun keine Flüssigkeit mehr strömt, weil beispielsweise keine Infusionslösung mehr vorliegt und/oder einem Patienten eine vollständige Dosis der Infusionslösung verabreicht wurde, kommt die Strömung durch die Kapillaren zum Erliegen und ein Flüssigkeitsniveau bleibt an einem oberen Ende der Kapillaren, d.h. die Filtermembran 8 bleibt im Wesentlichen vollkommen benetzt bzw. mit der Infusionslösung in Kontakt. Aufgrund von Kapillarkräften bildet sich, wie in Fig. 7b gezeigt, an den jeweiligen oberen Endabschnitten der einzelnen Kapillaren ein konkaver Meniskus bzw. eine konkave Wölbung an der Flüssigkeitsoberfläche. D.h. wenn die Strömung der Flüssigkeit stromauf der Filtermembran 8 stoppt, haftet die Flüssigkeit zwischen den Kapillaren fest und bildet so eine Barriere für Luft. Anders ausgedrückt, verhindert die Filtermembran 8 bei Erliegen der Strömung der Infusionslösung stromauf der Filtermembran 8 einen Lufteintritt in den Stromab-Abschnitt der Infusionsleitung 3. In Abhängigkeit des Durchmessers der Röhrchen 36 bzw. der Kapillaren, der Haftkraft zwischen der Flüssigkeit und der Oberfläche der Röhrchen 36 sowie der Oberflächenspannung (Oberflächenenergie) der Flüssigkeit, sind die Kapillarkräfte groß genug, um der Gewichtskraft der Flüssigkeit stromab entgegenzustehen und die Flüssigkeit am Einströmen in den Stromab-Abschnitt der Infusionsleitung 3 bzw. am Ausströmen aus dem Infusionsfilter 1 zu hindern. D.h. die Filtermembran 8 stellt eine Luftbarriere mittels kapillarem Flussstopp dar.

In dem bevorzugten Ausführungsbeispiel hat die Filtermembran 8 einen Blasendruck zwischen 0,2 und 0,45 bar und eine Fläche unter 2,0 cm², insbesondere unter 1,7 cm². Das Infusionsset 2 mit dem Infusionsfilter 1 gemäß dem bevorzugten Ausführungsbeispiel hat somit bei einer Druckdifferenz von 0,1 bar eine Flussrate über 120 ml/min, vorzugsweise 140 ml/min.

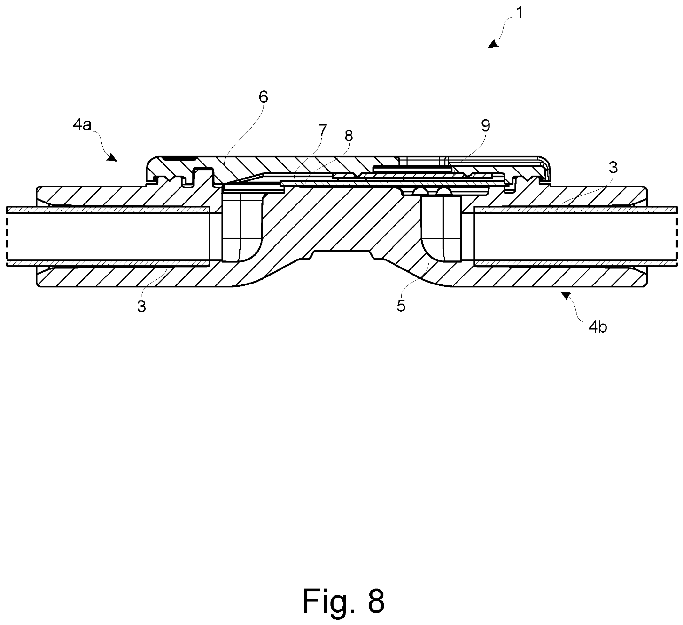

Der tellerförmige Belüftungsfilter 9 ist aus einem herkömmlichen, luftdurchlässigen Filtermaterial gefertigt und, wie in Fig. 8 zu erkennen, in dem Gehäusedeckel 6 aufgenommen. Eine Stirnfläche des Belüftungsfilters 9 ist dabei zu der Kavität 7 hin ausgerichtet und eine weitere Stirnfläche liegt an der Innenseite des Gehäusedeckels 6 an. In dem Gehäusedeckel 6 ist, wie vorstehend erwähnt, das Langloch 27 ausgebildet, über welches die Gehäuseinnenseite mit der Gehäuseaußenseite in Verbindung steht. Wie in Fig. 1 zu erkennen, ist der Belüftungsfilter 9 dabei so in dem Gehäusedeckel 6 aufgenommen, dass der Belüftungsfilter 9 das Langloch 27 vollständig abdeckt. Somit kann Luft, welche sich, wie nachstehend näher beschrieben, in der Kavität 7 sammelt, durch den Belüftungsfilter 9 und das Langloch 27 auf die Gehäuseaußenseite strömen. D.h. der Belüftungsfilter 9 und das Langloch 27 dienen zur Entlüftung des Infusionsfilters 1.

In Fig. 8 ist der Infusionsfilter 1 gemäß dem bevorzugten Ausführungsbeispiel im montierten Zustand mit eingeführter Infusionsleitung 3 gezeigt. Wenn nun die Infusionslösung über die Infusionsleitung 3 und den Gehäuseeinlass 12 in den Infusionsfilter 1 einströmt, tritt sie zunächst an der Schnittstelle, d.h. der oberflächenbündig mit dem Innendurchmesser der Infusionsleitung abschließenden Stufe 16, in den Infusionsfilter 1 ein und wird im Umlenkbereich 17 zur Kavität 7 hin umgelenkt, wo sie durch die Einlassöffnung 18 in die Kavität 7 einströmt. Die Schräge 35 am Gehäusedeckel 6 sorgt hier für eine turbulenzfreie Umlenkung und somit für eine optimale Anströmung/Benetzung der Filtermembran 8. Solange Infusionslösung vorhanden ist, strömt diese über die Filtermembran 8, d.h. durch die Röhrchen 36 und wird mit Hilfe der Rippen 23 und Noppen 24 hin zu der Auslassöffnung 19 geführt. Durch die Auslassöffnung 19 strömt die Infusionslösung hin zu dem Umlenkbereich 17, welcher die Strömung umlenkt, so dass die Infusionslösung über die Stufe 16 ohne Ablösung in den Stromab-Abschnitt der Infusionsleitung 3 geführt wird. D.h. entlang des gesamten Strömungspfads sind keine Stufen/Absätze ausgebildet, an welchen sich residuale Luftblasen festsetzen können. Zudem sind alle Umlenkungen als Rampen/Schrägen oder als Radien ausgeführt, so dass sich keine Totwassergebiete ausbilden und somit keine Turbulenzen und demzufolge auch keine Luftblasen in dem Infusionsfilter 1 gemäß dem bevorzugten Ausführungsbeispiel entstehen.

Wenn nun die Strömung der Infusionslösung zum Erliegen kommt, bleibt die Filtermembran 8, wie vorstehend beschrieben, vollständig benetzt und bildet so die Luftbarriere aus. Residuale Luft in den Strömungsabschnitten stromauf der Filtermembran 8 sammelt sich in der Kavität 7 und kann über den Belüftungsfilter 9 und das Langloch 27 ausströmen.

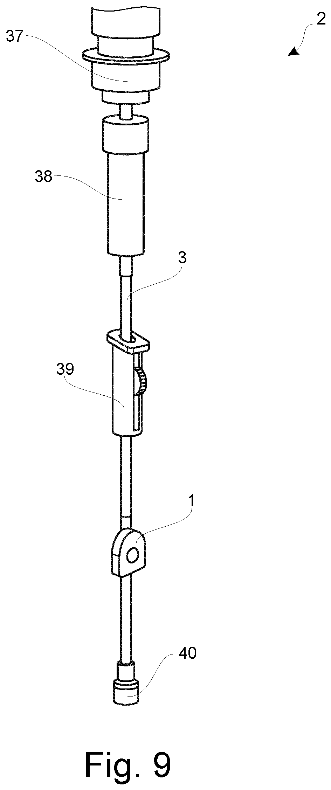

Fig. 9 zeigt das Infusionsset 2 mit der Infusionsleitung 3 und dem eingebauten Infusionsfilter 1 gemäß dem bevorzugten Ausführungsbeispiel. Stromauf des Infusionsfilters 1 ist ein Anschluss 37 für einen Behälter zur Aufnahme/Lagerung der Infusionslösung angeordnet. An den Anschluss 37 schließt sich eine Tropfkammer 38, welche eine erste Vorrichtung zur Luftabscheidung darstellt, und eine Schlauchklemme 39 zur Regelung der Strömung an. Stromab des Infusionsfilters 1 ist wiederum ein weiterer (Patienten-)Anschluss 40 angeordnet, welcher mit einer Injektionsstelle eines Patienten oder einer weiteren Infusionsleitung fluidisch verbunden werden kann. Anders ausgedrückt, strömt die Infusionslösung aus dem Behälter über den Anschluss 37 in die Infusionsleitung 3, durch die Tropfkammer 38, die Schlauchklemme 39, den Infusionsfilter 1 und den Anschluss 40. Die vorstehend beschriebene turbulenzfreie Durchströmung des Infusionsfilters 1 ermöglicht es, den Infusionsfilter 1 im Nahbereich des (Patienten-)Anschlusses 40 anzuordnen. Die Anordnung des Infusionsfilters 1 kann je nach erforderlicher Therapie an einer beliebigen Stelle in der Infusionsleitung 3 eingebaut sein. Auch ist es möglich, dass der Infusionsfilter 1 direkt im Anschluss 40 integriert ist.

In dem vorstehend beschriebenen, bevorzugten Ausführungsbeispiel ist die Filtermembran 9 aus dem Set an parallelen Röhrchen 11 aufgebaut. Alternativ ist jedoch auch eine Filtermembran 9 aus einem Block, welcher in Strömungsrichtung mit feinen Spalten durchzogen ist. Zwischen diesen Spalten kann bei Erliegen der Strömung die Flüssigkeit anhaften und sich so die Luftbarriere ausbilden. Zudem ist es auch denkbar, dass die Filtermembran 9 eine Vielzahl an Lamellen aufweist oder aus einem offenen porösen Material, wie beispielsweise einem Filz oder Schaum, gefertigt ist.

### Bezugszeichenliste

- 1: Infusionsfilter/Luftabscheider
- 2: Infusionsset
- 3: Infusionsleitung
- 4: Gehäuse
- 4a: stromaufwärtiger Gehäuseabschnitt
- 4b: stromabwärtiger Gehäuseabschnitt
- 5: Gehäuseunterteil
- 6: Gehäusedeckel
- 6a: erster Abschnitt
- 6b: zweiter Abschnitt
- 7: Kavität
- 8: Filtermembran
- 9: Belüftungsfilter
- 10: Hauptkörper
- 10a: erster Abschnitt
- 10b: zweiter Abschnitt
- 11: Balkenkörper
- 12: Gehäuseeinlass
- 13: Gehäuseauslass
- 14: Einführstruktur
- 15: Aufnahmebereich
- 16: Stufe
- 17: Umlenkbereich/Krümmer
- 18: Einlassöffnung
- 19: Auslassöffnung
- 20: Montageabschnitt
- 21: Montagezapfen
- 22: Steg
- 23: Rippe
- 24: Noppe
- 25: Steg
- 26: Schulterbereich
- 27: Langloch/Belüftungsöffnung
- 28: Steg
- 29: Schulterbereich
- 30: Montagezapfen
- 31: erster Steg
- 32: zweiter Steg
- 33: Montagebereich
- 34: Montageöffnung
- 35: Schräge
- 36: Röhrchen/Kapillare
- 37: Behälteranschluss
- 38: Tropfkammer
- 39: Schlauchklemme
- 40: Patientenanschluss

## Patentansprüche

1. Infusionsfilter (1) für eine oder einer medizinische(n) Infusionsleitung (3) mit einem Gehäuse (4), welches einen Strömungspfad zwischen einem jeweils mit einem Abschnitt der Infusionsleitung (3) koppelbaren Gehäuseeinlass (12) und Gehäuseauslass (13) definiert, und einer in dem Strömungspfad angeordneten, hydrophilen Filtermembran (8), die parallel zur von der Infusionsleitung (3) vorgegebenen Hauptströmungsrichtung ausgerichtet und derart im Gehäuse (4) platziert ist, dass sie das Gehäuse in einen stromaufwärtigen und einen stromabwärtigen Gehäuseabschnitt (4a, 4b) unterteilt,
**dadurch gekennzeichnet, dass** das Gehäuse (4) stromauf der Filtermembran (8) ein erstes Strömungsleitelement in Form eines Krümmers (17) für eine erste Umlenkung einer Strömung entlang des Strömungspfads quer, insbesondere senkrecht, zur Hauptströmungsrichtung und ein unmittelbar darauf folgendes zweites Strömungsleitelement in Form einer schräg zur Hauptströmungsrichtung ausgerichteten Prallfläche (35) für eine zweite Umlenkung der Strömung zurück in die Hauptströmungsrichtung aufweist, um so ein Anströmen der Filtermembran (8) ausschließlich an deren stromaufwärtigen Membranseite zu gewährleisten.

2. Infusionsfilter (1) nach Anspruch 1, **dadurch gekennzeichnet, dass** das Gehäuse (4) stromab der Filtermembran (8) ein drittes Strömungsleitelement in Form eines Krümmers (17) aufweist, welches die im Wesentlichen senkrecht durch die Filtermembran (8) strömende Strömung in die Hauptströmungsrichtung umlenkt.

3. Infusionsfilter (1) nach Anspruch 1 oder 2, **dadurch gekennzeichnet, dass** der Gehäuseeinlass (12) und der Gehäuseauslass (13) einen Aufnahmebereich (15) zur Aufnahme des jeweiligen Abschnitts der Infusionsleitung (3) aufweisen, und die Aufnahmebereiche (15) so an Dimensionen der Infusionsleitung (3) angepasst sind, dass im gekoppelten Zustand Innendurchmesser der Infusionsleitung (3) oberflächenbündig mit den Aufnahmebereichen (15) abschließen.

4. Infusionsfilter (1) nach einem der vorhergehenden Ansprüche 1 bis 3, ferner **gekennzeichnet durch** einen in dem Gehäuse (4), vorzugsweise dessen stromaufwärtigen Gehäuseabschnitt angeordneten, luftdurchlässigen Belüftungsfilter (9), welcher über eine in dem Gehäuse (4) ausgebildete Öffnung (27) mit einer Gehäuseaußenseite in Verbindung steht.

5. Infusionsfilter (1) nach einem der vorhergehenden Ansprüche 1 bis 4,
**dadurch gekennzeichnet, dass** das Gehäuse (4) eine Stützstruktur (22, 23, 24) zur Aufnahme oder Lagerung der Filtermembran (8) aufweist, welche einen der Geometrie der Filtermembran (8) folgenden, geschlossenen, vorzugsweise kreisrunden, Steg (22) und innerhalb dieses Stegs (22) angeordnete Unterstützungselemente, insbesondere Rippen (23) und Noppen (24), aufweist.

6. Infusionsfilter (1) nach Anspruch 5, **dadurch gekennzeichnet, dass** die Rippen (23) in Hauptströmungsrichtung ausrichtend stromab zur Filtermembran (8) unmittelbar dieser nachfolgend angeordnet sind und die Noppen (24) stromab zu den Rippen (23) sowie vorzugsweise bezüglich der Hauptströmungsrichtung querversetzt zu den Rippen (23) platziert sind.

7. Infusionsfilter (1) nach einem der vorhergehenden Ansprüche 1 bis 6,
**dadurch gekennzeichnet, dass** die Filtermembran (8) einen Blasendruck zwischen 0,2 und 0,45 bar und/oder eine zur Durchströmung bestimmte Fläche unter 2,0 cm², vorzugsweise unter 1,7 cm², hat.

8. Infusionsfilter (1) nach einem der vorhergehenden Ansprüche 1 bis 7,
**dadurch gekennzeichnet, dass** die Filtermembran (8) aus einer Vielzahl an parallelen Röhrchen (36), aus einem Block mit Spalten, aus Lamellen oder einem offenen porösen Material, insbesondere einer Membran, einem Filz oder Schaum, aufgebaut ist.

9. Infusionsset (2) mit einer Infusionsleitung (3), deren erster Endabschnitt einen Anschluss (37) für einen Behälter mit einer Infusionslösung und deren zweiter Endabschnitt einen Anschluss (40) für einen Patientenzugang oder eine weitere Infusionsleitung aufweist, und einem in der Infusionsleitung (3) angeordneten Infusionsfilter (1) nach einem der vorhergehenden Ansprüche 1 bis 8.

10. Infusionsset (2) nach Anspruch 9, **dadurch gekennzeichnet, dass** der Infusionsfilter (1) in Strömungsrichtung der Infusionslösung von dem ersten Endabschnitt zu dem zweiten Endabschnitt unmittelbar vor dem zweiten Endabschnitt angeordnet ist.

11. Infusionsset (2) nach Anspruch 9, **dadurch gekennzeichnet, dass** der Infusionsfilter (1) direkt in dem Anschluss (40) integriert ist.

## Claims

1. An infusion filter (1) for a or of a medical infusion line (3) comprising a housing (4) defining a flow path between a housing inlet (12) and a housing outlet (13) each coupleable to a portion of the infusion line (3), and a hydrophilic filter membrane (8) arranged in the flow path, which is aligned parallel to the main flow direction predetermined by the infusion line (3) and is placed in the housing (4) in such a way that it divides the housing into an upstream housing portion (4a) and a downstream housing portion (4b),
**characterized in that** the housing (4) has, upstream of the filter membrane (8), a first flow-guiding element in the form of an elbow (17) for a first deflection of a flow along the flow path transversely, in particular perpendicularly, to the main flow direction, and a second flow-guiding element directly following thereafter in the form of a baffle surface (35) oriented obliquely to the main flow direction for a second deflection of the flow back into the main flow direction, so as to ensure a flow against the filter membrane (8) exclusively at its upstream membrane side.

2. The infusion filter (1) according to claim 1, **characterized in that** the housing (4) has a third flow-guiding element downstream of the filter membrane (8) in the form of an elbow (17), which deflects the flow flowing substantially vertically through the filter membrane (8) into the main flow direction.

3. The infusion filter (1) according to claim 1 or 2, **characterized in that** the housing inlet (12) and the housing outlet (13) have a receiving region (15) for receiving the respective portion of the infusion line (3), and the receiving regions (15) are adapted to dimensions of the infusion line (3) in such a way that, in the coupled state, inner diameters of the infusion line (3) end flush with the surface of the receiving regions (15).

4. The infusion filter (1) according to one of the preceding claims 1 to 3, further **characterized by** an air-permeable vent filter (9) arranged in the housing (4), preferably its upstream housing portion, which is connected to an outer housing side via an opening (27) formed in the housing (4).

5. The infusion filter (1) according to one of the preceding claims 1 to 4, **characterized in that** the housing (4) has a support structure (22, 23, 24) for receiving or mounting the filter membrane (8), which has a closed, preferably circular, crosspiece (22) following the geometry of the filter membrane (8) and support elements, in particular ribs (23) and knobs (24), arranged within this crosspiece (22).

6. The infusion filter (1) according to claim 5, **characterized in that** the ribs (23) are arranged in the main flow direction aligned downstream of the filter membrane (8) directly following the latter and the knobs (24) are placed downstream of the ribs (23) and preferably transversely offset to the ribs (23) with respect to the main flow direction.

7. The infusion filter (1) according to one of the preceding claims 1 to 6, **characterized in that** the filter membrane (8) has a bubble pressure between 0.2 and 0.45 bar and/or an area intended for flow below 2.0 cm², preferably below 1.7 cm².

8. The infusion filter (1) according to one of the preceding claims 1 to 7, **characterized in that** the filter membrane (8) is constructed from a plurality of parallel tubes (36), from a block with gaps, from lamellae or from an open porous material, in particular a membrane, a felt or foam.

9. An infusion set (2) with an infusion line (3), the first end portion of which has a connector (37) for a container with an infusion solution and the second end portion of which has a connector (40) for a patient port or a further infusion line, and an infusion filter (1) arranged in the infusion line (3) according to one of the preceding claims 1 to 8.

10. The infusion set (2) according to claim 9, **characterized in that** the infusion filter (1) is arranged in the flow direction of the infusion solution from the first end portion to the second end portion directly upstream of the second end portion.

11. The infusion set (2) according to claim 9, **characterized in that** the infusion filter (1) is directly integrated in the connector (40).

## Revendications

1. Filtre de perfusion (1) pour une ou d'une ligne de perfusion (3) médicale avec un boîtier (4) qui définit un chemin d'écoulement entre une entrée de boîtier (12) et une sortie de boîtier (13) couplables respectivement avec une section de la ligne de perfusion (3), et une membrane de filtre (8) hydrophile agencée dans le chemin d'écoulement qui est orientée parallèlement au sens d'écoulement principal prédéfini par la ligne de perfusion (3) et placée dans le boîtier (4) de telle manière qu'elle divise le boîtier en une section de boîtier amont et une section de boîtier aval (4a, 4b),
**caractérisé en ce que** le boîtier (4) en amont de la membrane de filtre (8) présente un premier élément de guidage d'écoulement sous la forme d'un coude (17) pour un premier renvoi d'un écoulement le long du chemin d'écoulement transversalement, en particulier perpendiculairement au sens d'écoulement principal et un deuxième élément de guidage d'écoulement suivant directement sous la forme d'une surface de déflecteur (35) orientée en biais par rapport au sens d'écoulement principal pour un second renvoi de l'écoulement dans le sens d'écoulement principal afin de garantir ainsi un afflux de la membrane de filtre (8) exclusivement sur son côté de membrane amont.

2. Filtre de perfusion (1) selon la revendication 1, **caractérisé en ce que** le boîtier (4) en aval la membrane de filtre (8) présente un troisième élément de guidage d'écoulement sous la forme d'un coude (17) qui dévie l'écoulement s'écoulant sensiblement perpendiculairement par la membrane de filtre (8) dans le sens d'écoulement principal.

3. Filtre de perfusion (1) selon la revendication 1 ou 2, **caractérisé en ce que** l'entrée de boîtier (12) et la sortie de boîtier (13) présentent une zone de réception (15) pour la réception de la section respective de la ligne de perfusion (3) et les zones de réception (15) sont adaptées aux dimensions de la ligne de perfusion (3) de sorte que dans l'état couplé, des diamètres intérieurs de la ligne de perfusion (3) se terminent à fleur de surface avec les zones de réception (15).

4. Filtre de perfusion (1) selon l'une quelconque des revendications précédentes 1 à 3, de plus **caractérisé par** un filtre d'aération (9) perméable à l'air, agencé dans le boîtier (4), de préférence sa section de boîtier en amont, filtre qui est en liaison par le biais d'une ouverture (27) formée dans le boîtier (4) avec un côté extérieur de boîtier.

5. Filtre de perfusion (1) selon l'une quelconque des revendications précédentes 1 à 4, **caractérisé en ce que** le boîtier (4) présente une structure d'appui (22, 23, 24) pour la réception ou le logement de la membrane de filtre (8) qui présente une barrette (22) suivant la géométrie de la membrane de filtre (8), fermée de préférence ronde et circulaire et des éléments d'appui agencés dans cette barrette (22), en particulier des nervures (23) et des picots (24).

6. Filtre de perfusion (1) selon la revendication 5, **caractérisé en ce que** les nervures (23) sont agencées s'orientant dans le sens d'écoulement principal en aval de la membrane de filtre (8) suivant directement celle-ci et les picots (24) sont placés en aval des nervures (23) ainsi que de préférence en déport transversal des nervures (23) par rapport au sens d'écoulement principal.

7. Filtre de perfusion (1) selon l'une quelconque des revendications précédentes 1 à 6, **caractérisé en ce que** la membrane de filtre (8) présente une pression de bulle entre 0,2 et 0,45 bar et/ou une surface destinée à l'écoulement inférieure à 2,0 cm², de préférence inférieure à 1,7 cm².

8. Filtre de perfusion (1) selon l'une quelconque des revendications précédentes 1 à 7, **caractérisé en ce que** la membrane de filtre (8) est constituée d'une pluralité de petits tubes (36) parallèles, d'un bloc avec fentes, de lamelles ou d'un matériau poreux ouvert, en particulier d'une membrane, d'un feutre ou d'une mousse.

9. Ensemble de perfusion (2) avec une ligne de perfusion (3), dont la première section d'extrémité présente un raccord (37) pour un récipient avec une solution de perfusion et dont la seconde section d'extrémité présente un raccord (40) pour un accès patient ou une autre ligne de perfusion, et un filtre de perfusion (1) agencé dans la ligne de perfusion (3) selon l'une quelconque des revendications précédentes 1 à 8.

10. Ensemble de perfusion (2) selon la revendication 9, **caractérisé en ce que** le filtre de perfusion (1) est agencé dans le sens d'écoulement de la solution de perfusion de la première section d'extrémité à la seconde section d'extrémité directement avant la seconde section d'extrémité.

11. Ensemble de perfusion (2) selon la revendication 9, **caractérisé en ce que** le filtre de perfusion (1) est intégré directement dans le raccord (40).
